# EUROPEAN PATENT SPECIFICATION

(11) **EP 1 382 608 B1**
(45) Date of publication and mention of the grant of the patent: **31.08.2005**
(21) Application number: 02718617.0
(22) Date of filing: 19.04.2002
(51) Int. Cl.: C07D 519/06, A61K 31/546, A61P 31/04

(54) **SULFATE OF CEPHEM COMPOUND**
SULFAT VON CEPHEMVERBINDUNG
SULFATE D'UN COMPOSE CEPHEME

(30) Priority: 23.04.2001 JP 2001123732
(43) Date of publication of application: 21.01.2004
(73) Proprietor: SHIONOGI & CO., LTD., Osaka 541-0045 (JP)
(72) Inventor: ITANI, Hikaru, c/o Shionogi & Co., Ltd., Osaka-shi, Osaka 553-0002 (JP); IRIE, Tadashi, Suita-shi, Osaka 565-0854 (JP); MATSUBARA, F. c/o Shionogi & Co., Ltd., Osaka-shi, Osaka 553-0002 (JP); MYOJYO, H., c/o Shionogi & Co., Ltd., Osaka-shi, Osaka 553-0002 (JP)
(74) Representative: Vossius & Partner
(86) International application number: PCT/JP2002/003902
(87) International publication number: WO 2002/088147

(56) References cited:
- WO-A1-00/32606
- US-A- 4 609 653
- US-A- 4 734 408

## Description

### Technical Filed

The present invention relates to sulfates of a cefem compound, solvates, or crystals thereof, which are useful as medicines like antibacterial agents, as well as methods for preparation thereof.

### Background Art

In general, for the development of cefem compounds as medicines, especially injections, a compound of an extremely high quality is desired, preferably as an isolated crystal.

A cefem compound of the present invention is represented by the formula: 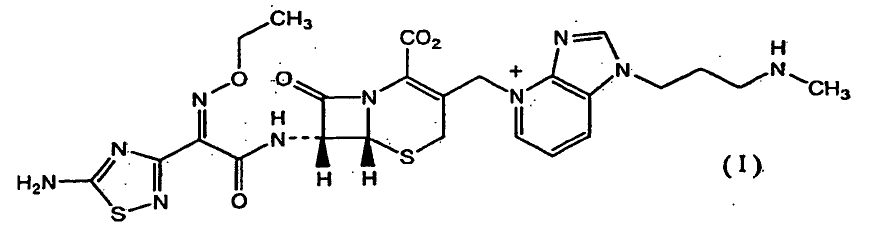 (hereinafter referred to as compound (I)). A corresponding hydrochloride, described in Example 6-2 of WO 00/32606, is isolated only as a non-crystal. Thus, crystallization of compound (I) has not been reported yet.

Therefore, in order to develop compound (I) as medicines, especially injections, it has been necessary to isolate the compound in high quality. Preferably, compound (I) or a salt thereof has been desired to be isolated as a more stable crystal.

### Disclosure of the Invention

The present inventors have intensively studied to find that isolation of compound (I) as sulfates or solvates thereof affords it in high quality including preservation stability, and that such a sulfate can be isolated as a stable crystal, whereby to accomplish the following present inventions.
(1) a sulfate or its solvate of the compound (I) of the formula: 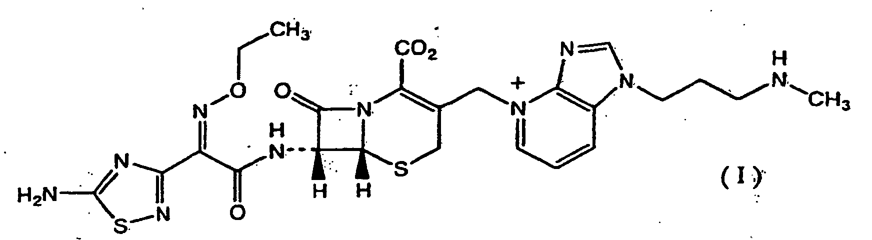
(2) a compound of above (1) which is a monosulfate hydrate.
(3) a compound of above (1) or (2) which is a monosulfate 1- to 7-hydrate.
(4) a compound of above (1) or (2) which is a monosulfate 1- to 5-hydrate.
(5) a compound of above (1) or (2) which is a monosulfate 1- to 3-hydrate.
(6) a crystal of the compound of any one of above (1) to (5).
(7) a crystal of above (6), which has an X-ray diffraction pattern having a primary peak at a spacing (d)=10.04, 9.65, 5.13, 4.51, 4.18, 3.58, 3.42, 3.35, and 2.98(Å).
(8) a crystal of above (7), which is a monosulfate 7-hydrate of compound (I).
(9) a crystal of above (6), which has an X-ray diffraction pattern having a primary peak at a spacing (d)=16.66, 9.52, 8.36, 7.26, 4.76, 4.55, 4.18, 3.67, 3.64, 3.61, 3.39, and 3.34 (Å).
(10) a crystal of above (9), which is a monosulfate 6-hydrate of compound (I)
(11) a crystal of above (6), which has an X-ray diffraction pattern having a primary peak at a spacing (d)= 9.75, 9.42, 4.56, 4.17, 3.69, 3.61, 3.41, and 3.34 (Å).
(12) a crystal of above (11), which is a monosulfate 5-hydrate of compound (I)
(13) a crystal of above (6), which has an X-ray diffraction pattern having a primary peak at a spacing (d)= 9.71, 9.36, 7.36, 4.99, 4.70, 4.55, 4.17, 3.92, 3.67, 3.61, and 3.40 (Å).
(14) a crystal of above (13), which is a monosulfate 4-hydrate of compound (I).
(15) a crystal of above (6), which has an X-ray diffraction pattern having a primary peak at a spacing (d)= 16.79, 9.80, 7.72, 5.75, 4.57, 4.19, 4.13, 3.69, 3.62, 3.42, 3.35 and 2.96 (Å).
(16) a crystal of above (15), which is a monosulfate 3-hydrate of compound (I).
(17) a crystal of above (6), which has an X-ray diffraction pattern having a primary peak at a spacing (d)= 16.66, 9.52, 8.32, 7.60, 4.58, 4.17, 4.08, 3.71, 3.62, 3.40, 3.33 and 2.95 (Å).
(18) a crystal of above (17), which is a monosulfate 1-hydrate of compound (I).
(19) a crystal of above (6), which has an X-ray diffraction pattern having a primary peak at (d)=16.60, 9.42, 4.54, 4.13, 3.59, 3.38, 3.31, 2.94 and 2.92 (A).
(20) a crystal of above (19), which is a monosulfate 2-hydrate of compound (I).
(21) a crystal of above (1), which has an X-ray diffraction pattern having a primary peak at (d)=7.70, 5.48, 5.12, 4.26, 3.93, 3.85, 3.53, 3.42, and 3.08 (A).
(22) a crystal of above (21), which is monosulfate anhydride of compound (I).
(23) a pharmaceutical composition containing a sulfate of compound(I), solvate, or crystal thereof described in any one of above (1) to (22) or a mixture thereof.
(24) a pharmaceutical composition of above (23) for use as an antibacterial agent.
(25) a pharmaceutical composition of above (23) for use as an injection.
(26) a method for preparing a sulfate of compound(I), solvate, or crystal thereof described in any one of above (1) to (22) or a mixture thereof, comprising a process of reacting a 0.5 sulfate of compound (I) of above (I) or solvate thereof with sulfuric acid.
(27) a 0.5 sulfate of compound (I) of above (I) or solvate thereof.
(28) a method for preparing a sulfate of compound(I), solvate, or crystal thereof described in any one of above (1) to (22) or a mixture thereof, comprising a process of reacting a monohydrochloride of compound (I) of above (I) with sulfuric acid.

### Brief Description of Drawings

(Fig. 1) This is a chart of X-ray diffraction pattern of the crystal obtained in Example 2. The vertical axis represents peak intensity (cps) and the horizontal axis represents diffraction angle 2θ (°).
(Fig. 2) This is a chart of X-ray diffraction pattern of a crystal obtained in Example 4.
(Fig. 3) This is a chart of X-ray diffraction pattern of a crystal obtained in Example 5.
(Fig. 4) This is a chart of X-ray diffraction pattern of a crystal obtained in Example 6(1).
(Fig. 5) This is a chart of X-ray diffraction pattern of a crystal obtained in Example 6(2).
(Fig. 6) This is a chart of X-ray diffraction pattern of a crystal obtained in Example 7.
(Fig. 7) This is a chart of X-ray diffraction pattern of a crystal obtained in Example 8.
(Fig. 8) This is a chart of X-ray diffraction pattern of a crystal obtained in Example 9.
(Fig. 9) This is a chart of X-ray diffraction pattern of a crystal obtained in Example 10.

### Best Mode for Carrying Out the Invention

In a sulfate of compound (I), the number of sulfuric acid is, not limited thereto, preferably 0.5 or 1, and more preferably 1. A 0.5 sulfate of compound (I) is useful also as an intermediate for a monosulfate.

The above sulfate may be a solvate and the solvent is exemplified by water, an organic solvent such as alcohol (e.g., ethanol, isopropanol, tetrahydrofuran, acetone, dioxane) or a mixture thereof.

The number of the above solvent depends on production method, preservation condition or the like, and the amount of an organic solvent is preferably little in a medicine. The solvate is preferably a hydrate and the number of water is preferably 1 to 7, more preferably 1 to 5, and most preferably 1 to 4 or 1 to 3. A sulfate of compound (I) is preferably monosulfate anhydride.

A sulfate of compound (I) or solvate thereof is a crystal or non crystal, preferably a crystal, and most preferably a crystal of monosulfate hydrate or a crystal of monosulfate anhydride. Those crystals are exemplified by those having an X-ray diffraction pattern having, at least, a primary peak at or around the following values, and such crystals are referred to as A type to H type crystals. While preferably being a single crystal having a specific X-ray diffraction pattern, a crystal of the present invention may be a mixture thereof.
A type: space (d)=10.04, 9.65, 5.13, 4.51, 4.18, 3.58, 3.42, 3.35, and 2.98 (Å)
B type: space (d)=16.66, 9.52, 8.36, 7.26, 4.76, 4.55, 4.18, 3.67, 3.64, 3.61, 3.39, and 3.34 (Å)
C type: space(d)=9.75, 9.42, 4.56, 4.17, 3.69, 3.61, 3.41, and 3.34 (Å)
D type: space(d)=9.71, 9.36, 7.36, 4.99, 4.70, 4.55, 4.17, 3.92, 3.67, 3.61, and 3.40 (Å)
E type: space(d)=16.79, 9.80, 7.72, 5.75, 4.57, 4.19, 4.13, 3.69, 3.62, 3.42, 3.35 and 2.96 (Å)
F type: space(d)=16.66, 9.52, 8.32, 7.60, 4.58, 4.17, 4.08, 3.71, 3.62, 3.40, 3.33 and 2.95 (Å)
G type: space(d)=16.60, 9.42, 4.54, 4.13, 3.59, 3.38, 3.31, 2.94 and 2.92 (Å)
H type: space(d)=7.70, 5.48, 5.12, 4.26, 3.93, 3.85, 3.53, 3.42, and 3.08 (Å) (X-ray detection condition: CuKα line (wave length λ=1.54 Å), tube voltage 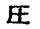 40kV, tube current 40mA; 2dsinθ=nλ (n is integer, θ is diffraction angle))

The above values of space (d) correspond to main X-ray peaks having strong relative intensity, thus a structure of each crystal can not always be determined by their selves. Namely, the other peak(s) may be involved in each X-ray diffraction pattern. When a crystal is measured by X-ray diffraction, measurement error may occur in peaks to some extent depending on a measurement apparatus, measurement condition or the presence of adhesion solvates etc. For example, a measurement error of about ±0.2 may occur in the value of space (d). Even when a very high-precious equipment is used, a measurement error of about ±0.01 to ± 0.1 may occur. Therefore, such measurement error should be considered in identifying each crystal structure. Any crystal, characterized by X-ray diffraction pattern substantially the same as shown above, is included within the scope of the present invention.

The above crystals may include the above mentioned solvates as a combined solvate or an adhesion solvate. Preferably, such a crystal is a hydrate optionally containing an organic solvent or anhydride. In a hydrate, the number of water is, not limited thereto, preferably 1 to 8 or 1 to 7, more preferably 1 to 5, and most preferably 1 to 4 or 1 to 3. In a preferred embodiment of the monosulfate crystal of compound (I), the above described A type can be 7 hydrate, B type can be 6 hydrate, C type can be 5 hydrate, D type can be 4 hydrate, E type can be 3 hydrate, F type can be 1 hydrate, and G type can be 2 hydrate. These crystals may contain a little amount of adhesion solvate depending on humidity, measuring condition or the like. On the other hand, H type crystal is preferably an anhydride.

A production of a sulfate of compound (I), solvate, or crystal thereof, or a mixture thereof may be, not limited thereto, carried out according to the following method.

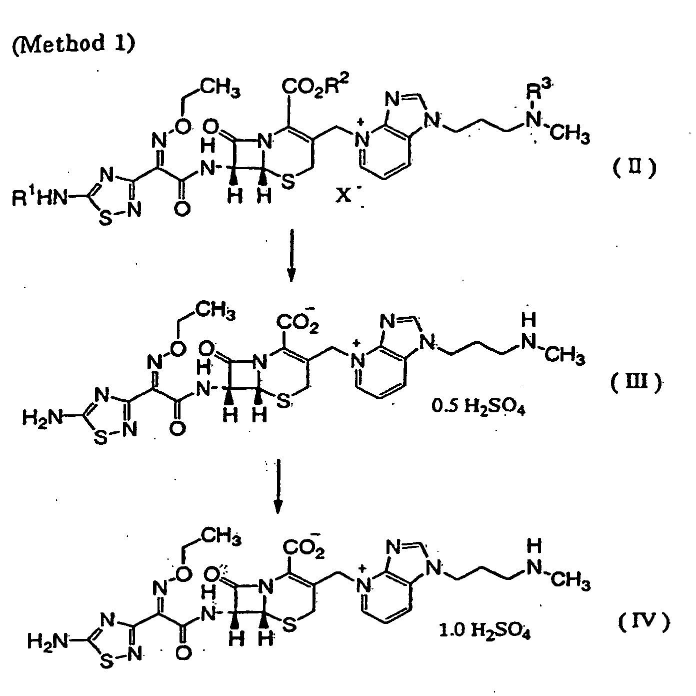

### (Process 1)

To an acidic solution of compound (II) (wherein R¹ is hydrogen or an amino-protecting group; R² is a carboxy-protecting group; R³ is hydrogen or an amino-protecting group; X is a counter ion such as halogen), a protected form of compound (I), which is preferably a solution of formic acid or acetic acid, is added dropwise under ice-cooling sulfuric acid (preferable concentration: about 60 to 98%) in an amount of about 10 to 30 mol equivalent, and preferably about 15 to 20 mol equivalent per compound (II) over several minutes to several ten minutes, and the mixture is stirred at the same temperature for several minutes to several hours The reaction mixture is poured into an organic solvent such as isopropanol, acetone, ethanol, or a mixture thereof, preferably which is cooled to about 0 to -20°C, so as to crystallize compound (III), 0.5 sulfate of compound (I). The starting material, compound (II), may be synthesized according to a method described in WO 00/32606. Substituents are preferably as that R¹ and R³ are amino-protecting groups (e.g., t-butoxycirbonyl), R² is a carboxy-protecting group (e.g., p-methoxybenzyl), and X is Cl, Br, or I.

In the present process, deprotection of compound (II) and formation of the sulfate can be accomplished by one reaction.

### (Process 2)

To an aqueous solution of compound (III), are added, preferably at a temperature of 0°C to room temperature, and more preferably about 3 to 10°C, an organic solvent (e.g., tetrahydrofuran) and sulfuric acid (preferable concentration: about 10 to 60%) which is preferably in an amount of about 0.5 to 1.0mol equivalent, and more preferably about 0.5 to 0.6mol equivalent per compound(III), so as to crystallize compound (IV), monosulfate of compound (I). When being hardly crystallized, compound (IV) can be treated as follows: an insoluble product is filtered off, then the filtrate is allowed to stand, to which a seed of the crystal may be added for crystallization. The precipitation may be dried to give a preferred crystal of compound (IV). The number of water combined with compound (IV), depending on crystallization condition, humidity, or drying condition, is for example 5 to 7.

While being useful as an intermediate for preparing a monosulfate of compound (I) as explained above, compound (III) may also be used as an active ingredient of a medicine.

### (Method 2)

A monohydrochloride of compound (I) is optionally treated with a base (e.g., NaOH), then reacted with sulfuric acid to give a monosulfate of compound (I).

Preferably, a monohydrochloride of compound (I) is reacted with sulfuric acid for several minutes to several hours. The reaction mixture is subjected to chromatography with sulfuric acid, then the fluid is preferably adjusted to pH 4 to 6 and filtered. The filtrate is concentrated in vacuum, then poured into alcohol to give a 0.5 sulfate of compound (I). The product is treated according to the above Process 2 to give a monosulfate of compound (I).

A sulfate of compound (I) or crystal thereof may be a hydrate. The number of the combined water can be controlled by varying the condition of recrystallization or drying.

A solvate for recrystallization is exemplified by water, an organic solvent (e.g., alcohol, acetone, tetrahydrofuran, dioxane) or a mixture thereof.

A general drying condition is, for example, as follows: temperature about 10 to 50°C, preferably about 20 to 40°C; pressure about 0 to 100 mmHg, preferably about 1 to 60 mmHg; and time about 1 min to 24 hr, preferably about 1 to 10 hr. Examples thereof are shown below.
(Ex. 1) A 7- to 8-hydrate crystal is dissolved in water under optional heating, then it is allowed to stand at about 0 to 10°C for several days, followed by optional stirring for several minutes to several hours. After further allowing to stand, the obtained crystal is dried in vacuum (e.g., about 10 to 20 mmHg, around room temperature, about 1 to 3 hr) to give a 5-hydrate. The 5-hydrate is dried in vacuum for long hours (e.g., about 10 to 20 mmHg, around room temperature, about 7 to 20 hr) to convert into a 4-hydrate.
(Ex. 2) A 7- to 8-hydrate crystal is dissolved in water under optional heating, then it is cooled to about 0 to 20°C. An organic solvent (e.g., tetrahydrofuran) is added thereto and the mixture is allowed to stand at about 3 to 20°C for several hr to several days, to which an organic solvent is added, optionally together with a little amount of sulfuric acid, to give a crystal. The obtained crystal is dried in vacuum (e.g., about 10 to 20mmHg, around room temperature, about 1 to 3 hr) to give a 6-hydrate. The 6-hydrate crystal is dried to convert into a 4- to 5-hydrate crystal.
(Ex. 3) A 6-hydrate crystal is dried in vacuum to convert into other hydrate crystals such as 3-hydrate (drying condition: about 15 to 18mmHg, around room temperature, about 4 to 5 hr), 1-hydrate (drying condition: about 2 to 4mmHg, around room temperature, about 3 to 5 hr), and 2-hydrate (drying condition: about 5 to 15 mmHg, around room temperature, about 1 to 5 hr).
(Ex. 4) A hydrate crystal can be converted into an anhydride crystal by drying under heating (e.g., about 80°C or more) or by drying in vacuum (e.g., about 1mmHg or less, around room temperature, about 2 hr or more).

The present invention further provides a pharmaceutical composition containing, as an active ingredient, a sulfate of compound (I), solvate or crystal thereof, or a mixture thereof. The pharmaceutical composition is preferably an antibacterial agent. Examples of the pharmaceutical composition include e.g., a tablet, a granule, a capsule, and an injection, and preferred is an injection. Further provided are a method for preventing or treating infection diseases, which comprises administering a sulfate of compound (I), solvate or crystal thereof, or a mixture thereof, and use of the same for preparing a pharmaceutical composition for preparing an antibacterial agent.

A sulfate of compound (I), solvate or crystal thereof has a high preservation stability and a so high solubility (>100 mg/ml) that it does not or hardly become cloudy when dissolved into water. These characteristics are remarkable compared with that of a corresponding hydrochloride or the like. Thus, a sulfate of compound (I), solvate or crystal thereof is especially suitable for an active ingredient of injections such as a powder-filled preparation or a freeze-dried preparation.

The above mentioned pharmaceutical composition may contain a pharmaceutically acceptable additive such as an excipient, a disintegrating agent, a solubilizing agent, an emulsifying agent, or a stabilizing agent. In particular, when the composition is used as an injection, a base for pH control (e.g., sodium carbonate, amino acid such as arginine) may be added thereto together with distilled water, a physiological saline solution etc.

The daily dose of a sulfate of compound (I), its solvate or crystal, or a mixture thereof, depending on the age or state of patients, the kind of diseases etc., is usually about 0.1 to 100 mg/kg, and preferably about 0.5 to 50 mg/kg, and may be administered orally or parenterally, if necessary, in 2 to 4 divisions.

### Examples

Examples and Experiments are shown below. The powder X-ray diffraction patterns are shown in Figures and the representative peak values are described in Tables. These are not to be construed to limit the scope of the present invention.

### (Abbreviation)

Me: methyl; Et: ethyl; Boc: t-butoxycarbonyl; PMB: p-methoxybenzyl; DMF: dimethylformamide; i-PrOH: isopropanol; THF: tetrahydrofuran; MeCN: acetonitrile

### Example 1 Synthesis of 0.5 sulfate

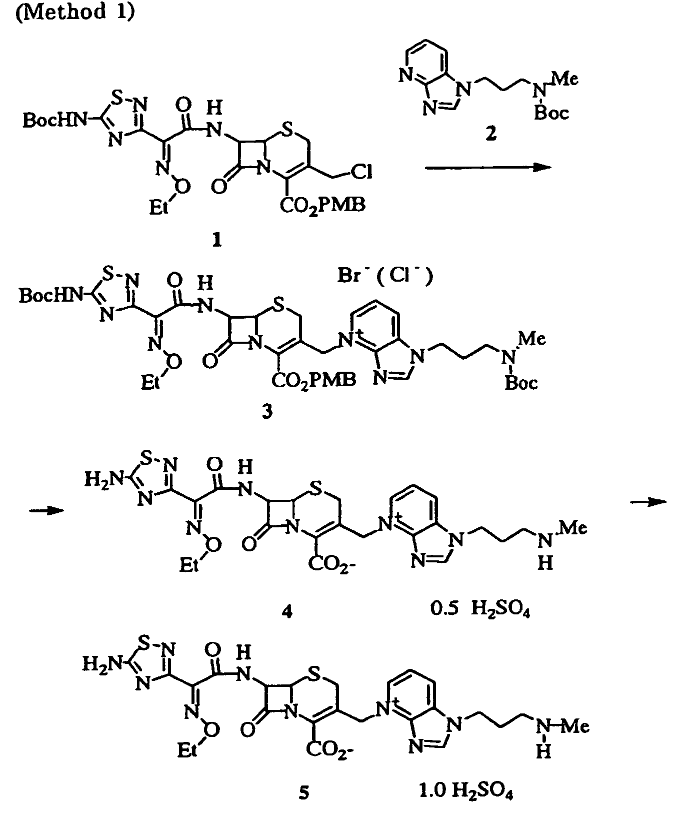

To a solution of imidazopyridine 2 (28.98 g, 99.81 mmol) and chloride 1 (81.2 g, purity 82%, 1eq) in DMF 92 ml, NaBr (20.58 g, 2eq) was added and the mixture was stirred at 5°C for 3.5 days. This suspension was poured into a 5% NaClaq. solution and the precipitated powder was filtered off, which was washed with brine and water successively, and concentrated in vacuum to give quaternary salt 3 as a mixture of Br salt and Cl salt (173.1 g).
Elementary Analysis for C₃₇H₄₅N₁₀O₈S₂C₁₀.2Br 0.8 H₂O
calculation: C : 48.78%, H : 5.20%, N : 15.38%, S : 7.04%, Cl : 0.78%, Br : 7.02%
measurement: C : 48.66%, H : 5.20%, N : 15.24%, S : 7.06%, Cl : 1.10%, Br : 7.14%

To a solution of compound 3 in 98% formic acid 220 ml, was added dropwise under ice-cooling 62% sulfuric acid 511 ml for 15 min. and the mixture was stirred at the same temperature for 1 hr. The mixture was poured into a solution of 9.5 L i-PrOH / acetone (8.5/1) cooled to -20°C and the precipitated powder was filtered off, which was washed with isopropanol and acetone successively, and concentrated in vacuum to give crude 4. The product was dissolved into water, subjected to HP-20SS Daiya ion exchange resin (Mitsubishi Chemical Corporation) with a solution of 0.001N H₂SO₄ to 0.001N H₂SO₄/MeCN (96/4) and about 8L eluate was collected. Poly(4-vinylpyridine) was added thereto for adjusting the pH to 4.5, followed by filtration, and the filtrate was concentrated in vacuum to be about 600 ml, then lyophilization gave 0.5 sulfate 4 (non-crystal, 31.3 g, yield about 43%).
Elementary Analysis for C₂₄H₂₈N₁₀O₅S₂·0.54H₂SO₄·4.6 H₂O
calculation: C,39.14 ; H,5.24 ; N,19.02 ; S,11.06
measurement: C,39.22 ; H,5.18 ; N,19.22 ; S, 10.88 (%)

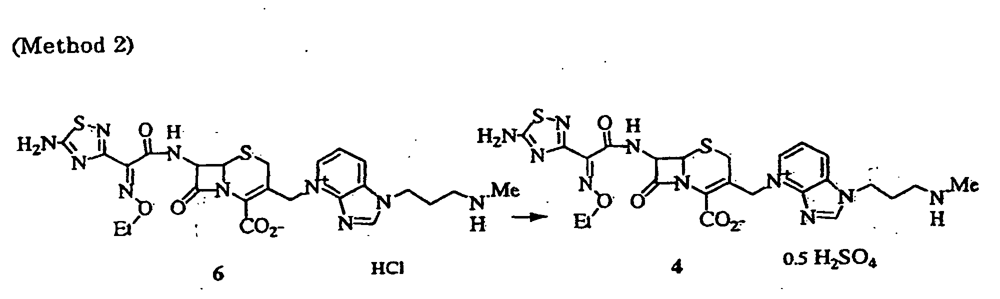

Hydrochloride 6 3.5 g described in Example 6-2 of WO 00/32606 was added to 2N H₂SO₄ 25 ml under stirring and ice-cooling and the mixture was stirred 15 min. The mixture was subjected to 250ml of HP-20SS DAIYA ION EXCHANGE RESIN (MITSUBISHI CHEMICAL CORPORATION) with 0.001N H₂SO₄ and the eluate was collected. Poly(4-vinylpyridine) was added thereto for adjusting the pH to 4.7, followed by filtration and concentration in vacuum. i-PrOH was added thereto and the precipitated powder was filtered off, then the filtrate was washed and concentrated in vacuum to give 0.5 sulfate 4.

### Example 2 Synthesis of monosulfate 7 hydrate crystal

To a solution of the above described compound 4 4 g (5.4 mmol) in 12 ml water, were added under cooling at 10°C 10N H₂SO₄ 0.65 ml and THF 13 ml, and a very small quantity of insoluble product was filtered off, allowing to stand for 1 week. The precipitated powder was filtered off and dried under reduced pressure of about 15 mmHg and at room temperature for 1.5 hr to give a monosulfate, compound 5 (1.67g) as 7 hydrate crystal.
Elementary Analysis for C₂₄H₂₈N₁₀O₅S₂·H₂SO₄·7 H₂O
calculation: C,34.95 ; H,5.38 ; N,16.98 ; S,11.66
measurement: C, 34.67 ; H, 5.30 ; N,17.16 ; S,11.72 (%)
¹H-NMR (D₂O) δ: 1.30(3H, t, J = 7.5 Hz), 2.42 (2H, m), 2.74 (3H, s), 3.16 (2H, t like, J =8.1 Hz),3.34 and 3.64 (2H, ABq, J = 18.3 Hz), 4.33 (2H, q, J = 7.5 Hz), 4.65 (2H, t like, J = 7.5 Hz), 5.25(1H, d, J = 4.8 Hz), 5.71 and 5.94 (2H, ABq, J = 15 Hz), 5.87 (1H, d, J = 4.8 Hz), 7.89(1H, dd, J = 8.1 Hz and 6.6 Hz), 8.82 (1H, d, J = 8.1 Hz), 8.86 (1H, d, J = 6.6 Hz), 8.89(1H, s).
mp(dec.): > 200 °C
powder X-ray: A type (ref.: Fig. 1 and Table 1)

**(Table 1)**

| 2φ(° ) | d(Å) | relative intensity (%) |
|---|---|---|
| 8.80 | 10.04 | 28 |
| 9.16 | 9.65 | 17 |
| 17.28 | 5.13 | 25 |
| 19.68 | 4.51 | 35 |
| 21.22 | 4.18 | 32 |
| 24.82 | 3.58 | 49 |
| 26.06 | 3.42 | 30 |
| 26.62 | 3.35 | 100 |
| 30.00 | 2.98 | 27 |

### Example 3 Recrystallization of monosulfate 7 hydrate

The monosulfate 7 hydrate crystal (20.6 g) of Example 2 was dissolved into water 78 ml under heating, to which acetone 59 ml and 1N H₂SO₄ 2.5 ml were added and a very small quantity of insoluble product was filtered off, then the filtrate was cooled to 13°C. Acetone 40 ml was added thereto, allowing to stand overnight. The solution was stirred at 4°C for 5 hr and allowed to stand overnight, then a crystal was filtered off. The crystal was washed with water/acetone, and dried under reduced pressure of about 15mmHg at room temperature for 2 hr to give a 7 hydrate crystal of compound 5 (12.7 g, about 62%). The X-ray peak of the crystal was equivalent to that of the crystal of Example 2.
KF water content for C₂₄H₂₈N₁₀O₅S₂·H₂SO₄·7.4 H₂O
calculation: 16.02 measurement: 16.05 (%).

### Example 4 Preparation of monosulfate 6 hydrate crystal

The monosulfate 7 hydrate 5.89 g of Example 3 was dissolved into distilled water for injection 24 ml under heating at 43°C, which was filtered through a microfilter washing with 6 ml water, and the filtrate was cooled to 17°C. THF 26 ml was added thereto, and the mixture was allowed to stand at 10°C for 22 hr and further at 4°C overnight. Further, THF 22 ml and a small amount of 2N H₂SO₄ were added thereto, allowing to stand overnight, then a crystal was filtered off. The crystal was washed with 2 ml ice-cooled solution of water/THF (1:1.6, 1:3) successively, and dried under reduced pressure of about 16mmHg at room temperature for 2 hr to give a monosulfate 6 hydrate crystal of compound (I) (4.73 g, 82%).
KF water content for C₂₄H₂₈N₁₀O₅S₂·H₂SO₄·6.2 H₂O
calculation: 13.78 measurement: 13.88 (%).
powder X-ray: B type (ref.: Fig. 2 and Table 2)

**(Table 2)**

| 2φ(° ) | d(Å) | relative intensity (%) |
|---|---|---|
| 5.30 | 16.66 | 3 |
| 9.28 | 9.52 | 6 |
| 10.58 | 8.36 | 6 |
| 12.18 | 7.26 | 9 |
| 18.64 | 4.76 | 5 |
| 19.50 | 4.55 | 5 |
| 21.26 | 4.18 | 32 |
| 24.20 | 3.67 | 12 |
| 24.46 | 3.64 | 13 |
| 24.64 | 3.61 | 12 |
| 26.24 | 3.39 | 7 |
| 26.64 | 3.34 | 100 |

### Example 5 Preparation of monosulfate 5 hydrate crystal

The monosulfate 7 hydrate 6.0 g of Example 3 was dissolved into 24 ml distilled water for injection under heating at 35°C, which was filtered through a microfilter washing with 2 ml water. A small amount of crystal seed was added to the filtrate, allowing to stand at 10°C for 3 days. The solution was stirred at 4°C for 3 hr, allowing to stand overnight and filtered to give a crystal. The crystal was washed with 1 ml cooled water and 1 ml water/EtOH (4:1, 2:1, 1:1, 1:2, 1:9) successively, and dried under reduced pressure of about 15 mmHg at room temperature for 1.5 hr to give a monosulfate 5 hydrate crystal of compound (I) 2.4 g.
KF water content for C₂₄H₂₈N₁₀O₅S₂·H₂SO₄·5 H₂O
calculation: 11.42 measurement: 11.46 (%).
¹³C-NMR (D₂O) δ : 16.59, 28.45, 28.84, 35.54, 46.24, 48.59, 57.27, 60.15, 61.59, 75.02, 119.29, 122.49, 131.81, 133.95, 134.98, 141.11, 148.97, 150.97, 153.50, 163.53, 166.64, 166.97, 168.87, 187.00.
solid ¹³C-NMR : outer standard: Hexamethyl benzene 817.3 ppm (The sample was prepared in dry N₂ atmosphere.) δ: 13.72, 16.29, 34.06, 44.94, 58.25, 73.64, 120.80, 124.73, 126.87, 128.23, 131.82, 140.66, 146.34, 148.77, 161.01, 166.20, 168.48, 185.04.
powder X-ray: C type (ref.: Fig. 3 and Table 3)

**(Table 3)**

| 2φ(° ) | d(Å) | relative intensity (%) |
|---|---|---|
| 9.06 | 9.75 | 24 |
| 9.38 | 9.42 | 18 |
| 19.44 | 4.56 | 25 |
| 21.28 | 4.17 | 40 |
| 24.08 | 3.69 | 30 |
| 24.62 | 3.61 | 28 |
| 26.14 | 3.41 | 20 |
| 26.68 | 3.34 | 100 |

### Example 6 Preparation of monosulfate 4 hydrate crystal

(1) The monosulfate 6 hydrate crystal 4.59 g obtained in Example 4 was dried under reduced pressure of 15 mmHg at room temperature for 21 hr to give a monosulfate 4 hydrate crystal of compound (I) 4.40 g.
KF water content for C₂₄H₂₈N₁₀O₅S₂·H₂SO₄·4.3 H₂O
calculation: 9.98, measurement: 9.98 (%).
solid ¹³C-NMR (The sample was prepared in dry N₂ atmosphere.) δ: 13.61, 24.74, 33.91, 45.08, 56.15, 58.29, 73.68, 120.69, 123.90, 126.81, 131.13, 140.31, 145.94, 148.81, 162.50, 166.10, 168.57, 184.84 .
IR (Nujol) (The sample was prepared in dry N₂ atmosphere.) cm⁻¹:
3346~3089, 2730, 2521~2457, 1880, 1759, 1687, 1633, 1585, 1554, 1518, 1267, 1215, 1147, 1061, 935, 818.
powder X-ray: D type (ref.: Fig. 4 and Table 4)

**(Table 4)**

| 2φ(° ) | d(Å) | relative intensity (%) |
|---|---|---|
| 9.10 | 9.71 | 28 |
| 9.44 | 9.36 | 43 |
| 12.02 | 7.36 | 30 |
| 17.78 | 4.99 | 31 |
| 18.80 | 4.70 | 30 |
| 19.50 | 4.55 | 48 |
| 21.30 | 4.17 | 100 |
| 22.64 | 3.92 | 34 |
| 24.22 | 3.67 | 61 |
| 24.66 | 3.61 | 72 |
| 26.16 | 3.40 | 52 |

(2) The monosulfate 5 hydrate 2.22 g obtained in Example 5 was dried under reduced pressure of 50 to 30 mmHg at room temperature for 2 hr and at 20 mmHg for 9.5 hr to give a monosulfate 4 hydrate crystal of compound (I).
KF water content for C₂₄H₂₈N₁₀O₅S₂·H₂SO₄·4.4 H₂O
calculation:10.19, measurement: 10.22 (%).
solid ¹³C-NMR: outer standard: Hexamethyl benzene 817.3 ppm (The sample was prepared in dry N₂ atmosphere.) δ : 13.66, 17.64, 19,24, 24.10, 32.60, 34,49, 45.27, 55.96, 58.68, 69.60, 73.24, 120.50, 124.09, 128.70, 130.35, 132.05, 139.24, 146.62, 149.05, 161.14, 162.70, 166.05, 168.57, 185.08 .
IR (Nujol) (The sample was prepared in dry N₂ atmosphere.) cm⁻¹: 3344~3029, 2733, 2524~2467, 1876, 1759, 1687, 1633, 1585, 1554, 1518, 1269, 1215, 1149, 1061, 937, 818.
mp(dec.): >200 °C
powder X-ray: D type (ref.: Fig. 5 and Table 5)

**(Table 5)**

| 2φ(° ) | d(Å) | relative intensity (%) |
|---|---|---|
| 9.08 | 9.73 | 10 |
| 9.40 | 9.40 | 9 |
| 12.04 | 7.34 | 4 |
| 17.72 | 5.00 | 10 |
| 18.84 | 4.71 | 12 |
| 19.48 | 4.55 | 13 |
| 21.30 | 4.17 | 28 |
| 24.20 | 3.67 | 10 |
| 24.66 | 3.61 | 15 |
| 26.20 | 3.40 | 11 |
| 26.70 | 3.34 | 100 |

### Example 7 Preparation of monosulfate 3 hydrate crystal

A monosulfate 6 hydrate crystal 8.50 g obtained according to the method of Example 4 was dried under reduced pressure of 16 mmHg at room temperature for 3.5 hr to give a monosulfate 3 hydrate crystal of compound (I) 7.94 g. The structure of the crystal was confirmed also by the single crystal X-ray analysis.
Elementary Analysis for C₂₄H₂₈N₁₀O₅S₂·H₂SO₄·3 H₂O
calculation: C,38.29; H, 4.82 ; N,18.61 ; S,12.78
measurement: C,38.14; H,4.84 ; N,18.64 ; S,12.66 (%)
KF water content:
calculation: 7.18, measurement: 7.77 (%).
solid ¹³C-NMR : outer standard: Hexamethyl benzene 817.3 ppm(The sample was prepared in dry N₂ atmosphere.) δ : 13.67, 17.65, 19,35, 23.82, 32.46, 34,45, 45.18, 55.92, 58.54, 69.76, 73.11, 120.17, 124.34, 128.95, 130.17, 132.01, 139.35, 149.11, 161.05, 166.01, 168.63, 184.90.
IR (Nujol) cm⁻¹. (The sample was prepared in dry N₂ atmosphere.) 3353, 3199, 2509, 1878, 1761, 1684, 1633, 1554, 1327, 1149, 1118, 1036, 937, 609.
powder X-ray: E type (ref.: Fig. 6 and Table 6) (The sample was prepared in dry N₂ atmosphere.)

**(Table 6)**

| 2φ(° ) | d(Å) | relative intensity (%) |
|---|---|---|
| 5.26 | 16.79 | 6 |
| 9.02 | 9.80 | 30 |
| 9.34 | 9.46 | 16 |
| 10.54 | 8.39 | 13 |
| 11.46 | 7.72 | 29 |
| 11.98 | 7.39 | 16 |
| 12.2 | 7.25 | 16 |
| 14.38 | 6.16 | 10 |
| 15.40 | 5.75 | 21 |
| 17.40 | 5.09 | 12 |
| 17.64 | 5.02 | 17 |
| 17.96 | 4.93 | 12 |
| 18.78 | 4.72 | 15 |
| 19.40 | 4.57 | 19 |
| 20.48 | 4.33 | 12 |
| 21.20 | 4.19 | 48 |
| 21.48 | 4.13 | 21 |
| 23.06 | 3.86 | 11 |
| 24.08 | 3.69 | 20 |
| 24.56 | 3.62 | 38 |
| 25.20 | 3.53 | 10 |
| 26.06 | 3.42 | 25 |
| 26.60 | 3.35 | 100 |
| 30.12 | 2.96 | 24 |

### Example 8 Preparation of monosulfate 1 hydrate crystal

A monosulfate 6 hydrate crystal 16.0 g obtained according to the method of Example 4 was dried under reduced pressure of 3 mmHg at 26°C for 4 hr to give monosulfate 1 hydrate crystal of compound (I) 14.15 g.
Elementary Analysis for C₂₄H₂₈N₁₀O₅S₂·H₂SO₄·1 H₂O
calculation: C,40.22; H,4.50 ; N,19.54 ; S,13.42
measurement: C,39.82; H,4.64 ; N,19.49 ; S,13.50 (%)
KF water content: calculation:2.51, measurement: 4.07 (%).
powder X-ray: F type (ref.: Fig. 7 and Table 7) (The sample was prepared in dry N₂ atmosphere.)

**(Table 7)**

| 2φ(° ) | d(Å) | relative intensity (%) |
|---|---|---|
| 5.30 | 16.66 | 11 |
| 9.28 | 9.52 | 29 |
| 10.62 | 8.32 | 21 |
| 11.64 | 7.60 | 17 |
| 19.36 | 4.58 | 16 |
| 21.30 | 4.17 | 46 |
| 21.74 | 4.08 | 18 |
| 23.94 | 3.71 | 20 |
| 24.58 | 3.62 | 32 |
| 26.24 | 3.40 | 20 |
| 26.72 | 3.33 | 100 |
| 30.28 | 2.95 | 21 |

solid ¹³C-NMR : outer standard: Hexamethyl benzene 817.3 ppm(The sample was prepared in dry N₂ atmosphere.) δ : 13.23, 17.07, 18.28, 25.03, 34.55, 46.35, 58.30, 73.84,116.96, 119.39, 125.99, 132.06, 134.44, 138.96, 147.26, 148.72, 160.76, 169.31, 184.17.

### Example 9 Preparation of monosulfate 2 hydrate crystal

A monosulfate 6 hydrate crystal 13.96 g obtained according to the method of Example 4 was dried under reduced pressure of 10 mmHg at 26°C for 3.75 hr to give monosulfate 2 hydrate crystal of compound (I) 12.72 g.
Elementary Analysis for C₂₄H₂₈N₁₀O₅S₂·H₂SO₄·2.1 H₂O
calculation: C,39.14; H,4.68 ; N,19.02 ; S,13.06
measurement: C,39.18; H,4.64 ; N,19.10 ; S,13.17 (%)
KF water content calculation:5.14, measurement: 5.19 (%).
solid ¹³C-NMR : outer standard:Hexamethyl benzene 817.3 ppm (The sample was prepared in dry N₂ atmosphere.) δ : 14.87, 16.91, 26.62, 34.88, 46.39, 58.34, 73.20, 120.35,121.56, 124.33, 129.19, 131.81, 139.19, 148.61, 160.90, 168.86, 184.74.
powder X-ray: G type (ref.: Fig. 8 and Table 8) (The sample was prepared in dry N₂ atmosphere.)

**(Table 8)**

| 2φ(° ) | d(Å) | relative intensity (%) |
|---|---|---|
| 5.32 | 16.60 | 35 |
| 9.38 | 9.42 | 31 |
| 19.52 | 4.54 | 25 |
| 21.52 | 4.13 | 49 |
| 24.78 | 3.59 | 36 |
| 26.32 | 3.38 | 36 |
| 26.94 | 3.31 | 100 |
| 30.38 | 2.94 | 24 |
| 30.54 | 2.92 | 34 |

### Example 10 Preparation of monosulfate anhydride crystal

The monosulfate 5.6 hydrate crystal 4.0g (KF water content 13%) obtained according to the method of Example 4 was dried under reduced pressure of 1mmHg or less at room temperature for 20 hr to give a monosulfate anhydride crystal of compound (I) 3.5 g.
KF water content calculation:0, measurement: 0.08 (%).
powder X-ray: H type (ref.: Fig. 9 and Table 9) (The sample was prepared in dry N₂ atmosphere.)

**(Table 9)**

| 2φ(°) | d(Å) | relative intensity (%) |
|---|---|---|
| 9.26 | 9.5425 | 21 |
| 11.48 | 7.7017 | 32 |
| 12.04 | 7.3447 | 14 |
| 12.78 | 6.9210 | 17 |
| 16.16 | 5.4803 | 22 |
| 17.28 | 5.1275 | 23 |
| 19.10 | 4.6428 | 16 |
| 19.50 | 4.5485 | 19 |
| 20.82 | 4.2630 | 29 |
| 21.60 | 4.1108 | 17 |
| 22.60 | 3.9311 | 29 |
| 23.10 | 3.8471 | 100 |
| 23.88 | 3.7232 | 21 |
| 24.68 | 3.6043 | 17 |
| 25.22 | 3.5283 | 28 |
| 26.06 | 3.4165 | 24 |
| 27.60 | 3.2292 | 17 |
| 28.98 | 3.0785 | 23 |

### Experiment 1

The crystals obtained in the above examples were filled into vials and the residue rate thereof was examined through the acceleration stability test at 40°C. Further, the turbidity of an aqueous solution containing the crystal was determined.

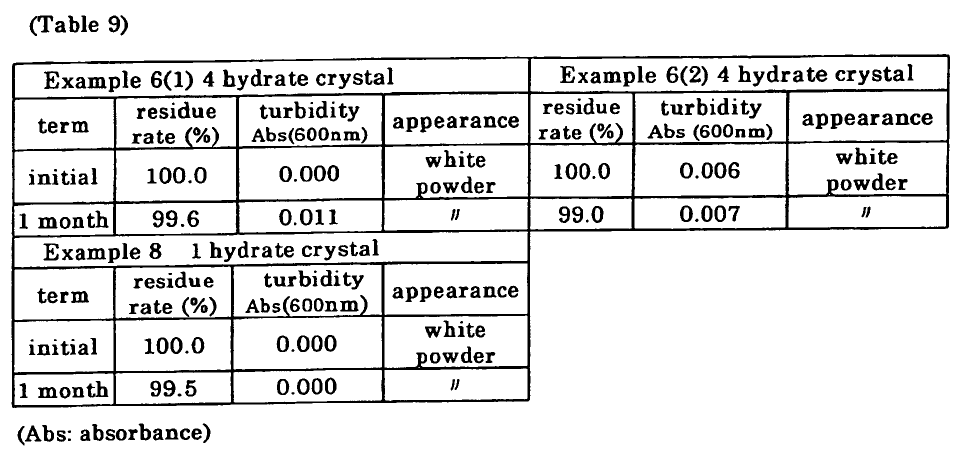

Sulfate crystals of the present invention exhibited a high stability for a long duration and the turbidity of an aqueous solution containing the crystal was hardly observed upon being dissolved into water.

### Experiment 2

The monosulfate crystal of compound (I) obtained in Example 9 was filled into vials and the residue rate thereof was examined through the acceleration stability test at 40°C. Further, the turbidity was determined.

As a reference compound, used was a lyophilization product of monohydrochloride of compound (I) (non-crystal, KF value: 1.7%, 0.6 H₂O) obtained according to the method of Example 6-2 of WO 00/32606.

**(Table 10)**

| | sulfate | | | | hydrochloride | | | |
|---|---|---|---|---|---|---|---|---|
| term | residue rate (%) | appeara nce of powder | turbidity Abs.(600n m) | appeara nce of solution | residue rate (%) | appearan ce of powder | turbidity Abs.(600n m) | appeara nce of solution |
| initial | 100.0 | white | 0.000 | colorless clarity | 100.0 | pale yellow | 0.001 | pale yellow clarity |
| 1 month | 99.7 | white | 0.000 | colorless clarity | 94.8 | pale yellow ∼ yellow | 0.565 | Yellow cloudy |
| 2 month | 99.0 | white | 0.000 | colorless clarity | 92.9 | pale yellow ∼ yellow | 1.338 | Yellow cloudy |

In comparison with the hydrochloride as a reference, a sulfate of the present invention exhibited a high stability for a long period without causing appearance change like discoloration. Further, the turbidity was not observed upon being dissolved into water. Thus, the sulfates of the present invention are more advantageous particularly for the use as injections than the corresponding hydrochloride.

### Experiment 3

The crystals obtained in Example 10 were filled into vials and the residue rate thereof was examined through the acceleration stability test at 40°C. Further, the turbidity of solutions was determined.

**(Table 11)**

| term | residue rate(%) | turbidity Abs(600nm) | appearance |
|---|---|---|---|
| initial | 100.0 | 0.002 | white powder |
| 1 month | 98.5 | 0.002 | " |
| (Abs: absorbance) | | | |

The sulfate anhydride crystal of the present invention exhibited a high stability for a long period and the turbidity was hardly observed upon being dissolved into water.

### Formulation Example 1

The monosulfate of compound (I) obtained in Example 7 and a base for adjusting pH were dissolved into distilled water to give an injection solution.

### Industrial Utility

Sulfates of the cefem compound of the present invention, solvates thereof or crystals of the same, possessing a high stability for a long period, are of extremely high quality. Thus, they can be used as an active ingredient of medicines such as an antibacterial agent, especially, an injection. Such compounds can be effectively prepared with a high yield and purity in a large scale by the production method of the present invention.

## Claims

1. A sulfate or its solvate of the compound (I) of the formula:

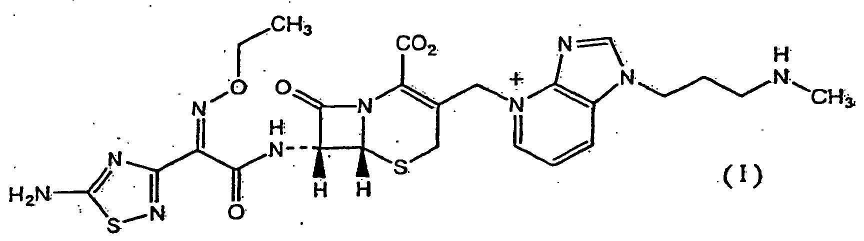

2. A compound of Claim 1 which is a monosulfate hydrate.

3. A compound of Claim 1 or 2 which is a monosulfate 1- to 7-hydrate.

4. A compound of Claim 1 or 2 which is a monosulfate 1- to 5-hydrate.

5. A compound of Claim 1 or 2 which is a monosulfate 1- to 3-hydrate.

6. A crystal of the compound described in any one of Claims 1 to 5.

7. A crystal of Claim 6, which has an X-ray diffraction pattern having a primary peak at a spacing (d)=10.04, 9.65, 5.13, 4.51, 4.18, 3.58, 3.42, 3.35, and 2.98 (Å).

8. A crystal of Claim 7, which is a monosulfate 7-hydrate of compound (I)

9. A crystal of Claim 6, which has an X-ray diffraction pattern having a primary peak at a spacing (d)=16.66, 9.52, 8.36, 7.26, 4.76, 4.55, 4.18, 3.67, 3.64, 3.61, 3.39, and 3.34 (Å).

10. A crystal of Claim 9, which is a monosulfate 6-hydrate of compound (I).

11. A crystal of Claim 6, which has an X-ray diffraction pattern having a primary peak at a spacing (d)= 9.75, 9.42, 4.56, 4.17, 3.69, 3.61, 3.41, and 3.34 (Å).

12. A crystal of Claim 11, which is a monosulfate 5-hydrate of compound (I).

13. A crystal of Claim 6, which has an X-ray diffraction pattern having a primary peak at a spacing (d)= 9.71, 9.36, 7.36, 4.99, 4.70, 4.55, 4.17, 3.92, 3.67, 3.61, and 3.40 (Å).

14. A crystal of Claim 13, which is a monosulfate 4-hydrate of compound (I).

15. A crystal of Claim 6, which has an X-ray diffraction pattern having a primary peak at a spacing (d)= 16.79, 9.80, 7.72, 5.75, 4.57, 4.19, 4.13, 3.69, 3.62, 3.42, 3.35 and 2.96 (A).

16. A crystal of Claim 15, which is a monosulfate 3-hydrate of compound (I).

17. A crystal of Claim 6, which has an X-ray diffraction pattern having a primary peak at a spacing (d)= 16.66, 9.52, 8.32, 7.60, 4.58, 4.17, 4.08, 3.71, 3.62, 3.40, 3.33 and 2.95 (Å).

18. A crystal of Claim 17, which is a monosulfate 1-hydrate of compound (I).

19. A crystal of Claim 6, which has an X-ray diffraction pattern having a primary peak at (d)=16.60, 9.42, 4.54, 4.13, 3.59, 3.38, 3.31, 2.94 and 2.92 (Å).

20. A crystal of Claim 19, which is a monosulfate 2-hydrate of compound (I).

21. A crystal of Claim 1, which has an X-ray diffraction pattern having a primary peak at (d)=7.70, 5.48, 5.12, 4.26, 3.93, 3.85, 3.53, 3.42, and 3.08 (Å).

22. A crystal of Claim 21, which is monosulfate anhydride of compound (I).

23. A pharmaceutical composition containing a sulfate of compound(I), solvate, or crystal thereof described in Claims 1 to 22 or a mixture thereof

24. A pharmaceutical composition of Claim 23 for use as an injection.

25. Use of a sulfate of compound (I), solvate or crystal thereof as described in anyone of claims 1-22 for the preparation of a pharmaceutical composition as an antibacterial agent.

26. A method for preparing a sulfate of compound (I), solvate, or crystal thereof described in any one of Claims 1 to 22 or a mixture thereof, comprising a process of reacting a 0.5 sulfate of compound (I) of Claim 1 or solvate thereof with sulfuric acid.

27. A 0.5 sulfate of compound (I) of Claim 1 or solvate thereof.

28. A method for preparing a sulfate of compound (I), solvate, or crystal thereof described in any one of Claims 1 to 22 or a mixture thereof, comprising a process of reacting a monohydrochloride of compound (I) of Claim 1 with sulfuric acid.

## Patentansprüche

1. Sulfat oder sein Solvat der Verbindung (I) der Formel:

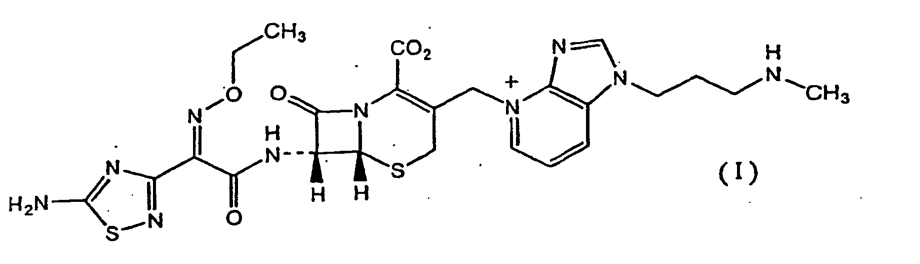

2. Verbindung nach Anspruch 1, die ein Monosulfat-Hydrat ist.

3. Verbindung nach Anspruch 1 oder 2, die ein Monosulfat-1- bis 7-Hydrat ist.

4. Verbindung nach Anspruch 1 oder 2, die ein Monosulfat-1- bis 5-Hydrat ist.

5. Verbindung nach Anspruch 1 oder 2, die ein Monosulfat-1- bis 3-Hydrat ist.

6. Kristall der Verbindung, die in einem der Ansprüche 1 bis 5 beschrieben ist.

7. Kristall nach Anspruch 6, der ein Röntgenbeugungsbild mit einem Primärpeak bei einem Abstand (d) = 10,04, 9,65, 5,13, 4,51, 4,18, 3,58, 3,42, 3,35 und 2,98 (Å) aufweist.

8. Kristall nach Anspruch 7, der ein Monosulfat-7-Hydrat der Verbindung (I) ist.

9. Kristall nach Anspruch 6, der ein Röntgenbeugungsbild mit einem Primärpeak bei einem Abstand (d) = 16,66, 9,52, 8,36, 7,26, 4,76, 4,55, 4,18, 3,67, 3,64, 3,61, 3,39 und 3,34 (Å) aufweist.

10. Kristall nach Anspruch 9, der ein Monosulfat-6-Hydrat der Verbindung (I) ist.

11. Kristall nach Anspruch 6, der einen Röntgenbeugungsbild mit einem Primärpeak bei einem Abstand (d) = 9,75, 9,42, 4,56, 4,17, 3,69, 3,61, 3,41 und 3,34 (Å) aufweist.

12. Kristall nach Anspruch 11, der ein Monosulfat-5-Hydrat der Verbindung (I) ist.

13. Kristall nach Anspruch 6, der ein Röntgenbeugungsbild mit einem Primärpeak bei einem Abstand (d) = 9,71, 9,36, 7,36, 4,99, 4,70, 4,55, 4,17, 3,92, 3,67, 3,61 und 3,40 (Å) aufweist.

14. Kristall nach Anspruch 13, der ein Monosulfat-4-Hydrat der Verbindung (I) ist.

15. Kristall nach Anspruch 6, der ein Röntgenbeugungsbild mit einem Primärpeak bei einem Abstand (d) = 16,79, 9,80, 7,72, 5,75, 4,57, 4,19, 4,13, 3,69, 3,62, 3,42, 3,35 und 2,96 (Å) aufweist.

16. Kristall nach Anspruch 15, der ein Monosulfat-3-Hydrat der Verbindung (I) ist.

17. Kristall nach Anspruch 6, der ein Röntgenbeugungsbild mit einem Primärpeak bei einem Abstand (d) = 16,66, 9,52, 8,32, 7,60, 4,58, 4,17, 4,08, 3,71, 3,62, 3,40, 3,33 und 2,95 (Å) aufweist.

18. Kristall nach Anspruch 17, der ein Monosulfat-1-Hydrat der Verbindung (I) ist.

19. Kristall nach Anspruch 6, der ein Röntgenbeugungsbild mit einem Primärpeak bei (d) = 16,60, 9,42, 4,54, 4,13, 3,59, 3,38, 3,31, 2,94 und 2,92 (Ä) aufweist.

20. Kristall nach Anspruch 19, der ein Monosulfat-2-Hydrat der Verbindung (I) ist.

21. Kristall nach Anspruch 1, der ein Röntgenbeugungsbild mit einem Primärpeak bei (d) = 7,70, 5,48, 5,12, 4,26, 3,93, 3,85, 3,53, 3,42 und 3,08 (Å) aufweist.

22. Kristall nach Anspruch 21, der ein Monosulfat-Anhydrid der Verbindung (I) ist.

23. Arzneimittel, enthaltend ein Sulfat der Verbindung (I), Solvat oder Kristall davon, wie in den Ansprüchen 1 bis 22 beschrieben, oder ein Gemisch davon.

24. Arzneimittel nach Anspruch 23 zur Verwendung als Injektion.

25. Verwendung eines Sulfats der Verbindung (I), Solvats oder Kristalls davon, wie in einem der Ansprüche 1 bis 22 beschrieben, zur Herstellung eines Arzneimittels als ein antibakterielles Mittel.

26. Verfahren zur Herstellung eines Sulfats der Verbindung (I), Solvats oder Kristalls davon, wie in einem der Ansprüche 1 bis 22 beschrieben, oder eines Gemischs davon, umfassend ein Verfahren zum Umsetzen eines 0,5-Sulfats der Verbindung (I) nach Anspruch 1 oder eines Solvats davon mit Schwefelsäure.

27. 0,5-Sulfat der Verbindung (I) nach Anspruch 1 oder ein Solvat davon.

28. Verfahren zur Herstellung eines Sulfats der Verbindung (I), Solvats oder Kristalls davon, wie in einem der Ansprüche 1 bis 22 beschrieben, oder eines Gemischs davon, umfassend ein Verfahren zum Umsetzen eines Monohydrochlorids der Verbindung (I) nach Anspruch 1 mit Schwefelsäure.

## Revendications

1. Sulfate ou son solvate du composé (I) de formule :

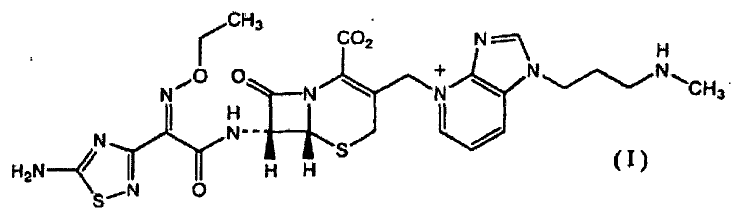

2. Composé selon la revendication 1 qui est un hydrate monosulfate.

3. Composé selon la revendication 1 ou 2 qui est un 1- à 7-hydrate monosulfate.

4. Composé selon la revendication 1 ou 2 qui est un 1- à 5-hydrate monosulfate.

5. Composé selon la revendication 1 ou 2 qui est un 1- à 3-hydrate monosulfate.

6. Cristal du composé décrit dans l'une quelconque des revendications 1 à 5.

7. Cristal selon la revendication 6, qui a un diagramme de diffraction aux rayons X présentant un pic primaire à un écartement (d) = 10,04, 9,65, 5,13, 4,51, 4,18, 3,58, 3,42, 3,35 et 2,98 (Å).

8. Cristal selon la revendication 7, qui est un 7-hydrate monosulfate du composé (I).

9. Cristal selon la revendication 6, qui a un diagramme de diffraction aux rayons X présentant un pic primaire à un écartement (d) = 16,66, 9,52, 8,36, 7,26, 4,76, 4,55, 4,18, 3,67, 3,64, 3,61, 3,39 et 3,34 (Å).

10. Cristal selon la revendication 9, qui est un 6-hydrate monosulfate du composé (I).

11. Cristal selon la revendication 6, qui a un diagramme de diffraction aux rayons X présentant un pic primaire à un écartement (d) = 9,75, 9,42, 4,56, 4,17, 3,69, 3,61, 3,41 et 3,34 (Å).

12. Cristal selon la revendication 11, qui est un 5-hydrate monosulfate du composé (I).

13. Cristal selon la revendication 6, qui a un diagramme de diffraction aux rayons X présentant un pic primaire à un écartement (d) = 9,71, 9,36, 7,36, 4,99, 4,70, 4,55, 4,17, 3,92, 3,67, 3,61 et 3,40 (Å).

14. Cristal selon la revendication 13, qui est un 4-hydrate monosulfate du composé (I).

15. Cristal selon la revendication 6, qui a un diagramme de diffraction aux rayons X présentant un pic primaire à un écartement (d) = 16,79, 9,80, 7,72, 5,75, 4,57, 4,19, 4,13, 3,69, 3,62, 3,42, 3,35 et 2,96 (Å).

16. Cristal selon la revendication 15, qui est un 3-hydrate monosulfate du composé (I).

17. Cristal selon la revendication 6, qui a un diagramme de diffraction aux rayons X présentant un pic primaire à un écartement (d) = 16,66, 9,52, 8,32, 7,60, 4,58, 4,17, 4,08, 3,71, 3,62, 3,40, 3,33 et 2,95 (Å).

18. Cristal selon la revendication 17, qui est un 1-hydrate monosulfate du composé (I).

19. Cristal selon la revendication 6, qui a un diagramme de diffraction aux rayons X présentant un pic primaire à un écartement (d) = 16,60, 9,42, 4,54, 4,13, 3,59, 3,38, 3,31, 2,94 et 2,92 (Å).

20. Cristal selon la revendication 19, qui est un 2-hydrate monosulfate du composé (I).

21. Cristal selon la revendication 1, qui a un diagramme de diffraction aux rayons X présentant un pic primaire à un écartement (d) = 7,70, 5,48, 5,12, 4,26, 3,93, 3,85, 3,53, 3,42 et 3,08 (Å).

22. Cristal selon la revendication 21, qui est un anhydride monosulfate du composé (I).

23. Composition pharmaceutique contenant un sulfate du composé (I), un solvate ou un cristal de celui-ci décrit dans l'une quelconque des revendications 1 à 22 ou un mélange de ceux-ci.

24. Composition pharmaceutique selon la revendication 23 pour utilisation par injection.

25. Utilisation d'un sulfate du composé (I), un solvate ou un cristal de celui-ci décrit dans l'une quelconque des revendications 1 à 22, pour la préparation d'une composition pharmaceutique destinée à être utilisée comme agent antibactérien.

26. Méthode pour préparer un sulfate du composé (I), un solvate ou un cristal de celui-ci décrit dans l'une quelconque des revendications 1 à 22 ou un mélange de ceux-ci, comprenant un procédé faisant réagir un 0,5 sulfate du composé (I) de la revendication 1 ou un solvate de celui-ci avec de l'acide sulfurique.

27. 0,5 sulfate du composé (I) de la revendication 1 ou un solvate de celui-ci.

28. Méthode pour préparer un sulfate du composé (I), un solvate ou un cristal de celui-ci décrit dans l'une quelconque des revendications 1 à 22 ou un mélange de ceux-ci, comprenant un procédé faisant réagir un monochlorhydrate du composé (I) de la revendication 1 avec de l'acide sulfurique.
